# EUROPEAN PATENT APPLICATION

(11) **EP 1 733 762 A1**
(43) Date of publication of application: **20.12.2006**
(21) Application number: 05011336.4
(22) Date of filing: 25.05.2005
(51) Int. Cl.: A61Q 11/00, A61K 8/24, A61K 8/19, A61K 8/23, A61K 8/46, A61K 8/41, A61K 8/49, A61K 8/27, A61K 8/40, A61K 8/34

(54) **Dental composition for detection of carious tissue, detection method**

(71) Applicant: 3M ESPE AG, 82229 Seefeld (DE)
(72) Inventor: Häberlein, Ingo, Dr., 82362 Weilheim (DE); Kappler, Oliver, Dr., 82362 Weilheim (DE); Guggenberger, Rainer, Dr., 82211 Herrsching (DE); Luchterhandt, Thomas, Dr., 86926 Greifenberg (DE); Hansen, Miriam, 81375 München (DE)
(74) Representative: Wallner, Astrid

(57) **Abstract**

The present invention relates to a composition comprising an aqueous solution of
1) at least one acidic compound,
2) at least one salt of said acidic compound.
3) at least one indicator that generates a detectable signal when said solution reaches a particular pH value that is less than 5.5

wherein 1) and 2) are selected such that the aqueous solution has a pH of less than that particular pH value where the indicator generates the signal.

It further relates to a method for detection of carious tissue on the surface of a tooth, comprising the steps of 1) providing the above mentioned composition, 2) applying said composition to the surface of said tooth, and 3) measuring the amount of time required for the composition to change color.

It further relates to the use of said composition to manufacture a remedy for detection of carious tissue.

## Description

The present invention relates to a dental composition. It more specifically relates to a composition for detection of carious tissue. It further relates to a method of detection of carious tissue.

### Background

Methods employed in the detection of carious tooth tissue are generally divided into physical and chemical detection methods. Known physical detection methods include use of x-rays, laser-enhanced fluorescence, trans-illumination with fiber optics as well as methods based on electrical conductivity of the tooth tissue. These methods are effective for detection of carious tissue in the overlying enamel area of teeth, but are not effective for detection of carious tissue in the underlying dentin. Physical methods described require auxiliary equipment and are time consuming as well.

Chemical methods for the detection of carious tooth tissue are also well known. Many are based on the ability of dyes, such as acid red or basic fuchsin, to stick to carious tooth tissue on contact. The method of using the acid red dyes include the steps of applying a solution of the dye to the tooth surface and removing any tissue that has turned red. This procedure is repeated until no color is detected after the dye solution is applied to the tooth. Use of such dyes and the aforementioned method to detect the presence of carious tooth tissue can result in removal of not only the carious tooth tissue, but also underlying healthy tooth tissue.

EP 0 865 785 A2 discloses an antimicrobial caries-detecting composition, which comprises water and/or a water-miscible solvent, a dye capable of staining the caries infected tissue, and at least one antimicrobial agent.

US 5,357,989 discloses a dental floss or tape impregnated with a pH sensitive dye that changes color in an acid environment.

US Patent No. 5,766,012 (Rosenbaum et al) describes an improved dental etchant composition comprising phosphoric acid as an active etchant and a colorant, wherein said colorant changes color at a pH of about 2.5 and wherein said etchant is a gel and contains from about 10% to about 20% by weight of phosphoric acid and about 0.001% to about 0.05% by weight of the colorant. A method is described for determining the correct amount of time for etching a caries-free tooth surface before applying a restorative to the tooth surface.

PCT publication no. WO 98/46194 (Hagne) describes an etching and detection composition for conditioning dental cavities in a single step, comprising a component a) which is an etching compound and a component b) which is a caries detector. The caries detector is selected from different dyes.

Ansari, Beeley, Reid and Foye (Journal of Oral Rehabilitation, 1999, 26; 453-458) determined that common dyes are not caries specific because they stain demineralized organic matrix and therefore cannot differentiate between infected unremineralizable tissue and reversibly decalcified tissue.

In addition, because caries causing bacteria and their metabolic products are present in carious lesions as well as in dental plaques, methods and materials that result in staining of caries causing bacteria and/or their metabolic products in addition to caries infected tissue, are not sufficiently accurate to identify and treat carious tissue.

It would therefore be desirable to provide a composition and a method for quick and reliable detection of carious tooth tissue that detects carious tooth tissue in both enamel and dentin tooth tissues.

In one aspect, the invention provides a composition useful for detecting caries comprising an aqueous solution of
1) at least one acidic compound,
2) at least one salt of said acidic compound,
3) at least one indicator that generates a detectable signal when said solution reaches a particular pH value that is less than 5.5
wherein 1) and 2) are selected such that the aqueous solution has a pH of less than that particular pH value where the indicator generates the signal.

In another aspect, the invention includes a method for detection of carious tissue on the surface of a tooth, comprising the steps of:
a) providing a composition comprising an aqueous solution of
   1) at least one acidic compound,
   2) at least one salt of said acidic compound,
   3) at least one indicator that generates a detectable signal when said solution reaches a particular pH value that is less than 5.5
   wherein 1) and 2) are selected such that the aqueous solution has a pH of less than that particular pH value where the indicator generates the signal,
b) applying said composition to the surface of said tooth, and
c) measuring the amount of time required for the composition to change color.

In another aspect the invention provides the use of a composition comprising an aqueous solution of
1) at least one acidic compound.
2) at least one salt of said acidic compound,
3) at least one indicator that generates a detectable signal when said solution reaches a particular pH value that is less than 5.5
wherein 1) and 2) are selected such that the aqueous solution has a pH of
less than that particular pH value where the indicator generates the signal, to manufacture a remedy for detection of carious tissue.

The composition according to the invention includes an indicator. The indicator may be selected from among many known indicator materials that can generate a detectable signal by itself or with another substance caused by pH-changes. Preferred are such indicators that generate an optical signal. This indicator may include but not be limited to materials that will change color, fluoresce at a particular pH or that will emit a signal detectable in the UV range. Preferred is a pH indicator that changes color. This color change should occur below a pH of 5.5.

The pH of the aqueous solution can be varied by selection of the type and the amount of the acidic compound(s) and the salt(s) of said acidic compound(s) used to prepare the aqueous solution. However, that acidic compound(s) and salt(s) in solution should yield a solution with a pH below the pH value or pH range where the indicator changes color.

The indicator can be one compound or a mixture of compounds. Preferably, the indicator changes color at a particular pH between 1.0 to 5.5. Preferably, the indicator and the aqueous solution are selected such that the composition has a specific color at a first pH and does not change color at decreasing pH values but changes color at increasing pH values.

One advantage of the present invention is that it changes color, as opposed to acting as a permanent dye that stains a demineralised organic matrix. Thus, sound tissues will not be stained.

Another advantage of the present invention is that the indicator changes color independently from the presence of bacteria. In other words, it does not change color as a result of a pH decrease that happens in the bacterial environment by the discharge of acidic metabolism products but it changes from its initial color to another color at increasing pH values.

Examples of suitable indicators include compounds selected from the group consisting of brilliant green, eosin yellow, erythrosin, methyl green, methyl violet, picric acids, cresol red, crystal violet; 2,4-dinitrophenol, 4-(dimethylamino)-azobenzol; bromphenol blue, sodium salt of bromphenol blue, congo red, methyl orange, bromcresol green, sodium salt of bromcresol green, m-cresol purple, thymol blue, sodium salt of thymol blue, p-xylenol blue; 2.2',2'''.4,4'-pentamethoxytriphenylcarbinol, eosin blue and "chinaldinrot" or mixtures thereof.

Particularly preferred indicators can be selected from the group consisting of m-cresol purple, thymol blue, sodium salt of thymol blue, p-xylenol blue; 2,2',2"', 4,4' pentamethoxytriphenylcarbinol; eosin blue and "chinaldinrot" or mixtures thereof.

Typically, the composition of the invention will comprise indicator in amounts of 50 ppm to 5 weight-%, preferably from 50 ppm to 1 weight-% and most preferably from 50 ppm to 0.1 weight-%.

In a preferred embodiment of the invention the composition further comprises an auxiliary colorant. This auxiliary colorant is particular advantageous if the indicator is a color changing indicator. The auxiliary colorant is one or more compounds that do not change color upon a pH change of the composition. Instead, the auxiliary colorant provides the composition of the invention with a first color that will improve detection of the color change of the indicator. For example, the auxiliary colorant can provide a background color to enhance the appearance of the color change.

### Auxiliary colorants useful in the composition include but are not limited to:

Blue colorants such as Patent Blue V. Patent Blue E 131, Erioglaucine, NEOZAPON blue 807 (commercially available from BASF, Ludwigshafen, Germany), methylene blue (commercially available from Hoechst), indigo carmine, indigo cartine (E 132), anthocyan (E 163);

Yellow colorants such as kurkumin (E 100), lactoflavin (E 101), riboflavin-5-phosphate (E 101a), Tatrazin (E 102), chinolin yellow (E 104), yellow orange S (E 110);
Red colorants such as cohenille (e 120), azorubin (E 122), amaranth (E 123), erythrosine (E 127), allura red (E 129) and ruby pigments (E 180).

Preferred auxiliary colorants are blue. More preferred auxiliary colorants are selected from the group consisting of erioglaucine, Patent Blue V and EOZAPON blue 807. Most preferred auxilliary colorant is erioglaucine.
Typically, amounts of auxiliary colorant of from 5 ppm to 1 weight-%, preferably from 10 ppm to 1000 ppm and most preferably from 50 ppm to 500 ppm, can be used to prepare the compositions of the invention.

The composition may further comprise one or more biochemical substances suitable for removing carious tissue. This includes, for example, one or a mixture of enzymes capable of removing carious tissue such as those described in WO 2004/017988 which disclosure is herein incorporated by reference.

If a 2 component solution as described in WO 2004/017988 is used, the indicator or the indicator together with the auxiliary colorant can be added for example to component A. This component A may further comprise one or more biochemical substances. The acidic compound according to this invention is incorporated in component B and the salt of said acidic compound is incorporated in component A and B. After combination with the component B of the 2 component solution in a ratio of, for example, 1:3 (A:B) the pH value of the resulting composition is in the range claimed with this invention.

In this case, the inventive composition of this embodiment may be applied to tissue that is clearly identified as carious. The composition removes a first part or all of the carious tissue through the biochemical activity. Simultaneously, the composition of the invention will indicate, by a fast color change, that carious tissue still is present. If all carious tissue has been removed, the color change appears very slow. Then, the practitioner can decide whether a further caries removal procedure is necessary or not. If necessary, he can apply the composition of the invention again or carry out other adjacent caries removing steps.

The acidic compound preferably is selected from the group comprising inorganic acids including oxy acids of phosphorus, chlorine, sulfur or nitrogen; organic acids including acetic acid, oxalic acid an other carboxylic acids; or mixtures thereof. Preferred are oxy acids of sulphur, phosphorus and nitrogen and most preferred is phosphoric acid.

Typically, the amount of acidic compound used in the composition of the invention is from 0.01 weight-% to 40 weight-%, preferably from 0.1 weight-% to 10 weight-% and most preferably from 0.5 weight-% to 5 weight-%, according to the total composition weight.

The acidic compound and the salt of said acidic compound together form a buffer system, so any salt can be used that forms a buffer with the respective acidic compound. For example, if phosphoric acid is employed as the acidic compound, the salt NaH₂PO₄ can be employed.
The amount of buffer used in the invention depends upon the amount of the acidic compound used and the desired pH of the composition.. For example, if a pH of approximately 2.1 is desired, the following combination can be used to create one litre of solution:

### Example of combination:

| | | |
|---|---|---|
| Water | | 1000g |
| NaH₂PO₄*2H₂O | | 156.25g |
| H₃PO₄ | | 45 g |

The composition of the invention may further comprise one or more additives such as rheology modifiers, stabilizers, fillers and the like. The rheology modifiers thicken the composition so that it remains on the surface of the tooth. Rheology modifiers may include, for example, inorganic materials such as fumed silica, quartz and glass having particle sizes of less than 1 µm. In particular materials having particle sizes in the range of 10 to 500 nm are desirable. Rheology modifiers can also include organic materials capable of thickening aqueous solutions, such as poly(meth)acrylic acid and its salts; polysaccharides such as gum arabic and cellulose derivatives such as hydroxyethylcelluloses, carboxymethyl celluloses; hydroxyethyl starches, xanthanates and alginates.
Generally, the compositions of the invention comprise additives in the amount of 0 weight-% to 30 weight-%, preferably from 2 weight-% to 15 weight-% and most preferably from 3 weight-% to 13 weight-% based on total composition weight.

Other, optional additives can include stabilizers as preservatives such as benzoic acids, benzoic acid esters usually in an amount of 50 ppm to 5 weight-%.

Furthermore fillers such as glasses, quartz and Zirconica silicates, with a particle size of above 0.6 µm can be added to the composition. These filler may be present in an amount of 1 weight-% to 70 weight-%, preferably from 5 weight-% to 40 weight-% and most preferably from 10 weight-% to 40 weight-%.

The composition of the invention can be used for the detection of carious tissue on the surface of a tooth, using a method comprising the steps of
a) providing a composition comprising an aqueous solution of at least one acidic compound, at least one salt of said acidic compound and at least one indicator that generates a detectable signal when said solution reaches a particular pH value that is less than 5.5, wherein the acidic compound and the salt of that acidic compound are selected such that the aqueous solution has a pH of less than that particular pH value where the indicator generates the signal.,
b) applying said composition to the surface of said tooth, and
c) measuring the amount of time required for the composition to change color.

In the method, a relatively rapid color change of the composition will indicate the presence of carious tissue. Typically, color changes dectectable within a minute and more preferably within 30 seconds are desirable. Without being bound this theory, one explanation for the color change is that caries dental tissue with collagen fibers is deteriorated by caries infections. The deteriorated tissue may release phosphate anions or other anions present in the dental tissue raising the pH in the region of the caries lesion. The aforementioned composition applied to the area of the carious lesion will detect this pH change and change color. The faster the color change the higher and/or faster the release of anions from the deteriorated tissue and the more likely there is a caries infection. If there is no caries infection, the composition will very gradually change color as it equilibrates with the pH of the health oral environment (which is at a higher pH than the composition), Color changes occurring over a longer time range such as over one minute would therefore, indicate that probably there is no caries infection. Thus, the dental practitioner will decide whether caries removal is necessary or not.

Preferably, the color change occurs in less than 1 minute, and preferably in less than 30 seconds, if caries infected tooth tissue is present..

The present invention also relates to a method of producing a composition comprising the steps of preparing a solution by dissolving an acidic compound, a salt of said acidic compound and a compound that changes color in deionized water wherein said acidic compound is selected that the aqueous solution has a pH of less than 5.5 and wherein said color changing compound changes color when said solution reaches a certain pH wherein said pH is less than 5.5.

The composition of the above described invention can be used to manufacture a remedy for detection of carious tissue.

It further can be used to manufacture a remedy for detection and removal of carious tissue.

### Example 1

An aqueous solution was prepared by combining 90 ml of deionized water, 4.5 ml phosphoric acid (60 %), 15.6125 g NaH₂PO₄ and 0.07 g sodium salt of thymol blue (Aldrich Chemical Company). Deionized water was added until the total volume was 100 ml. The pH of the solution was then adjusted with H₃PO₄ (60%) to a pH of 2.1. The composition appeared red.

A carious human tooth was treated with 1-3 drops (depending on the size of the cavity) of the red solution prepared above. The solution was agitated with a small brush for 20 seconds. After 15 second, the red color disappeared.

The composition was flushed from the tooth surface with water. The carious tooth mass was then removed using a rose bur.
The composition was then reapplied to the tooth surface and agitated with a small brush as before. The composition remained red for more than 60 seconds, indicating that no carious tissue was present and therefore sufficient tooth tissue had been removed.

### Example 2

An aqueous solution was prepared by combining 90 ml of deionized water, 4.5 ml phosphoric acid (60 %). 15.6125 g Na H₂ PO₄, 0.07 g sodium salt of thymol blue (Aldrich) and 0.01 g Patent Blue V (Aldrich), also known as acid blue v, C.l. food blue 3, Molecular formula: C₅₄ H₆₂ Na ₂ N₄ O₁₄ S _{4.} CAS No: 129-17-9. Deionized water was added until the total volume was 100 ml. The pH of the solution was then adjusted with H₃PO₄ (60%) to a pH of 2.1. The composition appeared to be purple.

A carious human tooth was treated with 1-3 drops (depending on the size of the cavity) of the purple solution prepared above. The solution was agitated with a small brush for 20 seconds. When the composition was applied to the carious tooth tissue, the solution turned green in ca. 15 seconds.
After carious tooth tissue had been removed as described in Example 1, the composition was reapplied to the tooth surface and the tooth retained its original color for more than one minute.

### Example 3

An aqueous solution was prepared by combining 90 ml of deionized water. 4.5 ml phosphoric acid (60 %), 1.600 g NaH₂PO₄ and 0.10 g sodium salt of bromphenol blue (Merck AG, Darmstadt, Germany). Deionized water was added until the total volume was 100 ml. The pH of the solution was then adjusted with 2.0 M NaOH to a pH of 2.9. The composition appeared yellow.
A carious human tooth was treated with 1-3 drops (depending on the size of the cavity) of the yellow solution prepared above. The solution was agitated with a small brush for 20 seconds. After ca. 20 seconds, the yellow color changed to a greenish-blue.
The composition was flushed from the tooth surface with water. The carious tooth mass was then removed using a rose bur.
The composition was then reapplied to the tooth surface and agitated with a small brush as before. The composition remained red for more than 60 seconds, indicating that no carious tissue was present and therefore sufficient tooth tissue had been removed.

### Example 4:

Two aqueous solutions were prepared. For component A a solution was prepared with deionized water comprising 0.9 % of pepsin. 1.2 % NaH₂PO₄, 0.5 % hydroxyethylcellulose (high viscosity), 0.07 % methylparahydroxybenzoate and 0.02 % propylparahydroxybenzoate and 700 ppm thymol blue. The pH value of the solution was adjusted to 5.5 by using NaOH in an amount of less than 0.1 %.

For component B a solution was prepared with deionized water comprising 15.6 % NaH₂PO₄, 0.5 % hydroxyethylcellulose (high viscosity), 4 % polyethylene glycol 200. The pH value of the solution was adjusted to 2 by using H₃PO₄ in an amount of less than 6 %.

Before use the component A and the component B were mixed in a ratio of 1 part component A : 3 parts component B.

### Example 5:

Two aqueous solutions were prepared. For component A a solution was prepared with deionized water comprising 0.9 % of pepsin, 1.2 % NaH₂PO₄, 0.5 % hydroxyethylcellulose (high viscosity), 0.07 % methylparahydroxybenzoate and 0.02 % propylparahydroxybenzoate and 700 ppm thymol blue and 100 ppm of patent blue. The pH value of the solution was adjusted to 5.5 by using NaOH in an amount of less than 0.1 %.

For component B a solution was prepared with deionized water comprising 15.6 % NaH₂PO₄, 0.5 % hydroxyethylcellulose (high viscosity), 4 % polyethylene glycol 200. The pH value of the solution was adjusted to 2 by using H₃PO₄ in an amount of less than 6 %.

Before use the component A and the component B were mixed in a ratio of 1 part component A : 3 parts component B.

## Claims

1. A composition comprising an aqueous solution of
1) at least one acidic compound,
2) at least one salt of said acidic compound,
3) at least one indicator that generates a detectable signal when said solution reaches a particular pH value that is less than 5.5
wherein 1) and 2) are selected such that the aqueous solution has a pH of less than that particular pH value where the indicator generates the signal.

2. A composition according to claim 1
wherein the indicator or the mixture of indicators generate a detectable signal when the solution reaches a particular pH value in the pH range from pH 1.0 to pH 5.5.

3. A composition according to any of the preceding claims
wherein the indicator is selected from the group consisting of brilliant green, eosin yellow, erythrosin, methyl green, methyl violet, picric acids, cresol red, crystal violet; 2,4-dinitrophenol, 4-(dimethylamino)-azobenzol; bromphenol blue, sodium salt of bromphenol blue, congo red, methyl orange, bromcresol green, sodium salt of bromcresol green, m-cresol purple, thymol blue, sodium salt of thymol blue, p-xylenol blue; 2,2',2"',4,4'-pentamethoxytriphenylcarbinol, eosin blue and "chinaldinrot" or mixtures thereof.

4. A composition according to any of the preceding claims,
comprising an auxiliary colorant.

5. A composition according to any of the preceding claims,
comprising one or more biochemical substances suitable for removing carious tissue.

6. A composition according to claim 5,
wherein the composition is a two component system.

7. A composition according to any of the preceding claims,
wherein one or more of said acidic compound is selected from the group consisting of the oxy acids of sulfur, phosphorus and nitrogen.

8. A composition according to any of the preceding claims,
wherein said acidic compound is phosphoric acid.

9. A composition according to any of the preceding claims
comprising one or more additives selected from the group consisting of rheology modifiers, stabilizers and fillers.

10. Method for detection of carious tissue on the surface of a tooth, comprising the steps of:
a) providing a composition according to any of the claims 1-9,
b) applying said composition to the surface of said tooth, and
c) measuring the amount of time required for the composition to change color.

11. Method according to claim 10,
wherein the color change appears within less than 1 minute if carious tooth tissue is present and the color change appears within 1 to 10 minutes if no carious tooth tissue is present.

12. Method according to claim 10,
wherein the color change appears within less than 30 seconds if carious tooth tissue is present and the color change appears within 1 to 5 minutes if no carious tooth tissue is present.

13. Method of preparing a composition for detection of carious tissue on the surface of a tooth, comprising the steps of preparing a solution by dissolving an acidic compound, a salt of said acidic compound and a compound that changes color in deionized water wherein said acidic compound is selected that the aqueous solution has a pH of less than 5.5 and wherein said color changing compound changes color when said solution reaches a certain pH wherein said pH is less than 5.5.

14. Use of a composition according to any of the preceding claims to manufacture a remedy for detection of carious tissue.
